(19) Europäisches Patentamt
European Patent Office
Office européen des brevets

(11) Veröffentlichungsnummer: **0 093 338 A1**

(12) # EUROPÄISCHE PATENTANMELDUNG

(21) Anmeldenummer: **83103906.0**

(22) Anmeldetag: **21.04.83**

(51) Int. Cl.³: **C 07 C 120/00**
C 07 C 121/38, C 07 C 121/75

(30) Priorität: **04.05.82 DE 3216569**

(43) Veröffentlichungstag der Anmeldung:
**09.11.83 Patentblatt 83/45**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Findeisen, Kurt, Dr.**
**In der Follmühle 10**
**D-5068 Odenthal 2(DE)**

(54) **Verfahren zur Herstellung von Cyanhydrincarbonsäureestern.**

(57) Die vorliegende Erfindung betrifft ein Verfahren zur Herstellung von Cyanhydrincarbonsäureestern der Formel (I)

```
      O    CN
  2   ‖    |    1
R  -  C-O- C -R          (I)
           |
           R
```

in welcher

R für Wasserstoff, gegebenenfalls durch Halogen, CN, Alkoxy oder Akoxycarbonyl substituiertes Alkyl oder Aryl steht,

$R^1$ für Wasserstoff, gegebenenfalls durch Halogen, CN, Alkoxy, Alkoxycarbonyl substituiertes Alkyl, Cycloalkyl oder Alkenyl, sowie für Aryl oder Heteroaryl steht und $R^1$ und $R^2$ gemeinsam für gegebenenfalls durch Halogen, CN, Alkyl substituiertes Alkylen stehen,

$R^2$ für gegebenenfalls substituiertes Alkyl, Cycloalkyl, Aryl oder Heteroaryl steht,

indem man Acylcyanide oder O-Acyl-tartronsäurenitrile (dimere Acylcyanide) oder Gemische der beiden Verbindungen, mit Aldehyden oder Ketonen in Gegenwart wasserfreier Basen gegebenenfalls in Gegenwart von Verdünnungsmitteln umsetzt.

EP 0 093 338 A1

Croydon Printing Company Ltd.

0093338



BAYER AKTIENGESELLSCHAFT 5090 Leverkusen, Bayerwerk

Zentralbereich
Patente, Marken und Lizenzen Rt/bc/c

IVa ZP

Verfahren zur Herstellung von Cyanhydrincarbonsäureestern

Die vorliegende Erfindung betrifft ein Verfahren zur Herstellung von Cyanhydrincarbonsäureestern.

Es ist bereits bekannt geworden, Cyanhydrincarbonsäureester herzustellen, indem man Acylcyanide mit Aldehyden in Gegenwart wäßriger Basen oder in cyanidionenhaltigen wäßrigen Lösungsmitteln umsetzt (Am. Chem. Soc. 71, 34 (1949); Angew. 68, 425-448 (1956)).

Die dort beschriebenen Reaktionen befriedigten nicht, da gute Ausbeuten nur mit sauberen Ausgangsmaterialien erreicht werden und sie außerdem auf bekannte Verbindungsklassen beschränkt sind.

Es wurde nun gefunden, daß Cyanhydrincarbonsäureester der allgemeinen Formel (I)

$$R^2-\overset{O}{\overset{\|}{C}}-O-\underset{R}{\overset{CN}{C}}-R^1 \qquad (I)$$

in welcher

R für Wasserstoff, gegebenenfalls durch Halogen, CN, Alkoxy oder Alkoxycarbonyl substituiertes Alkyl oder Aryl steht,

$R^1$ für Wasserstoff, gegebenenfalls durch Halogen, CN, Alkoxy, Alkoxycarbonyl substituiertes Alkyl, Cycloalkyl oder Alkenyl, sowie für Aryl oder Heteroaryl steht und $R^1$ und $R^2$ gmeinsam für gegebenenfalls durch Halogen, CN, Alkyl substituiertes Alkylen stehen,

$R^2$ für gegebenenfalls substituiertes Alkyl, Cycloalkyl, Aryl oder Heteroaryl steht,

erhalten werden, indem man Acylcyanide der Formel (II)

$$R^2-\overset{\overset{O}{\|}}{C}-CN \qquad \text{(II)}$$

in welcher

$R^2$ die oben angegebene Formel hat,

oder O-Acyl-tartronsäurenitrile (dimere Acylcyanide) der Formel (III)

$$R^2-\overset{\overset{O}{\|}}{C}-O-\underset{\underset{CN}{|}}{\overset{\overset{CN}{|}}{C}}-R^2 \qquad \text{(III)}$$

in welcher

$R^2$ die oben angegebene Bedeutung hat,

oder Gemische der Verbindungen der Formel (II) und (III) mit Aldehyden oder Ketonen der Formel (IV)

$$O=\underset{\underset{R}{|}}{C}-R^1 \qquad \text{(IV)}$$

in welcher
$R^1$ und R die oben angegebene Bedeutung haben,

in Gegenwart wasserfreier Basen gegebenenfalls in Gegenwart von Verdünnungsmitteln umsetzt.

Es war bekannt, das Acylcyanide, vor allem aliphatische und cycloaliphatische Acylcyanide, in Gegenwart von Basen rasch zu den O-Acyl-tartronsäurenitrilen (Formel III) dimerisieren. Es konnte nicht erwartet werden, daß sich diese O-Acyl-tartronsäurenitrile mit Aldehyden oder Ketonen in Gegenwart von Basen glatt und in guter Ausbeute zu den Cyanhydrincarbonsäureestern weiter umsetzen lassen. Es war zu erwarten, daß Ausbeute und Reinheit der gewünschten Produkte darunter leiden, daß die Verbindungen der Formel (III) verseift werden oder die Aldehyde oder Ketone der Formel (IV) Kondensationsreaktionen eingehen.

Für diese Annahme spricht auch, daß in Anwesenheit wäßriger Basen allein die Umsetzung nur zu ca. 49 % abläuft (Am. Chem. Soc. 71, 34 (1949)). Erst bei Zusatz von Alkalicyaniden läßt sich die Ausbeute auf 90 % steigern (Angew. Chemie 68, 425-448 (1956)).

Außerdem sind die bekannten Umsetzungen nur bei Benzoylcyanid und Benzaldehyd beschrieben. Es geht aus den Literaturstellen aber nichts darüber hervor, daß diese Reaktion allgemein anwendbar ist.

Die vorliegende Erfindung zeigt, daß die Umsetzung von Acylcyaniden und/oder ihren Dimeren mit Aldehyden oder Ketonen in sehr breitem chemischem Umfang erfolgen kann, wenn anstelle wäßriger Basen oder Alkalicyaniden wasserfreie Basen verwendet werden. Es war überraschend, daß dabei unabhängig von der Art der Ausgangsmaterialien die Cyanhydrincarbonsäureester der Formel (I) in guten Ausbeuten und Reinheiten hergestellt werden können, obwohl aus Angew. Chem. 68, S. 433 (1956) bekannt war, daß solche Reaktionen nur in Anwesenheit von Cyan-Ionen ablaufen.

Verwendet man α-Benzoyloxy-α-phenylmalonsäuredinitril und p-Chlor-Benzaldehyd als Ausgangsstoffe, so kann der Reaktionsablauf durch das folgende Formelschema wiedergegeben werden:

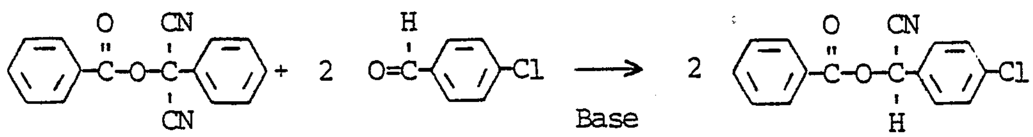


Verwendet man Benzylcyanid und Aceton als Ausgangsstoffe, so kann der Reaktionsablauf durch das folgende Formelschema wiedergegeben werden:

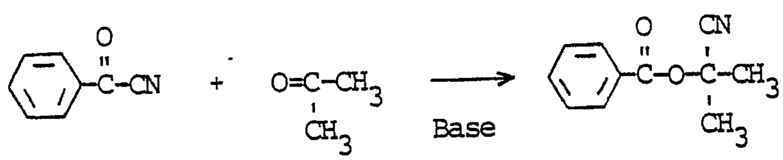

Die als Ausgangsstoffe verwendbaren Acylcyanide der Formel (II) sind bekannt oder können nach an sich bekannten Methoden hergestellt werden (Angew. Chemie 94, S. 1-86 (1982) und die darin angegebenen Literaturstellen).

Bevorzugt werden Acylcyanide der Formel (II) eingesetzt, in welcher

$R^2$ für $C_{1-6}$-Alkyl, das gegebenenfalls durch CN, Halogen, Alkoxy, Halogenalkoxy, $C_{1-4}$-Alkoxycarbonyl, Aryl, insbesondere gegebenenfalls durch Alkyl, Halogen, Alkoxy, Halogenalkoxy, Halogenalkyl substituiertes Phenyl substituiert ist, ferner für $C_{3-8}$-Cycloalkyl, das gegebenenfalls durch $C_{1-4}$-Alkyl, das gegebenenfalls durch Halogen substituiert ist, Styryl, das gegebenenfalls durch Halogen substituiert ist, $C_{2-6}$-Alkenyl, das gegebenenfalls durch Halogen substituiert ist, ferner für Aryl, insbesondere Phenyl, das gegebenenfalls durch Halogen, $C_{1-6}$-Alkyl, $C_{1-4}$-Halogenalkyl, $C_{1-4}$-Alkoxy, $C_{1-4}$-Halogenalkoxy, Methylendioxy, Ethylendioxy, die gegebenenfalls durch Halogen substituiert sind, Nitro, CN, $C_{1-4}$-Alkoxycarbonyl substituiert ist, ferner für einen 5- bis 6-gliedrigen N-, O- oder S-haltigen Heterocyclus der gegebenenfalls durch Alkyl, Halogenalkyl, Alkenyl oder Halogen substituiert ist und gegebenenfalls mit einem Benzolring annelliert sein können, steht.

Besonders bevorzugte Acylcyanide sind solche der Formel (II) in welcher

$R^2$ für geradkettiges oder verzweigtes $C_{1-4}$-Alkyl, $C_{2-4}$-Alkenyl, die gegebenenfalls substituiert sind, durch $C_{1-4}$-Alkoxy, $C_{1-5}$-Carbalkoxy, Halogen, insbesondere Fluor oder Chlor, CN oder Phenyl, das gegebenenfalls durch Chlor, Fluor, Brom, CN, $C_{1-4}$-Alkyl, $C_{1-2}$-Halogenalkyl, $C_{1-4}$-Alkoxy, $C_{1-4}$-Halogenalkoxy substituiert ist, steht, ferner für Cyclopropyl, Cyclobutyl, Cyclopentyl, Cyclohexyl, Cyclohexenyl, die gegebenenfalls substituiert sind durch $C_{1-4}$-Alkyl, insbesondere Methyl, $C_{2-4}$-Alkenyl, $C_{2-4}$-Halogenalkenyl, $C_{1-4}$-Halogenalkyl, Styryl, das gegebenenfalls durch Halogen, insbesondere Fluor, Chlor, Brom substituiert ist, ferner für Phenyl oder Naphthyl, die gegebenenfalls durch $C_{1-4}$-Alkyl, $C_{1-4}$-Alkoxy, $C_{1-4}$-Carbalkoxy, Nitro, CN, Halogen, insbesondere Chlor, Brom, Fluor substituiert wird, steht und ferner für gegebenenfalls durch Halogen wie Fluor, Chlor, Brom, $C_{1-4}$-Alkyl, $C_{1-4}$-Alkoxy substituierte Heterocyclen aus der Reihe Morpholin, Imidazol, Pyrazol, Pyrrol, Isoxazol, Piperidin, Oxazol, Triazol, Thiadiazol, Furan, Benzimidazol, steht.

Ganz besonders bevorzugt werden Acylcyanide der Formel (II) eingesetzt in welcher

$R^2$ für Phenyl, das gegebenenfalls durch Halogen wie Chlor, $C_{1-4}$-Alkyl wie Methyl, Ethyl oder CN,

substituiert ist, für $C_{1-6}$-Alkyl, das gegebenenfalls durch Halogen substituiert ist, steht.

Als Beispiele für Acylcyanide der Formel (II) seien genannt:

Acetylcyanid, Propionylcyanid, Butyrylcyanid, Pivaloylcyanid, Stearoylcyanid, Palmitinoylcyanid, Oleylcyanid, Cyclopropancarbonsäurecyanid, α-(2-(2,2-Dichlorovinyl)-3,3-dimethylcycloprop-1-yl)-α-oxoacetnitril, α-(2-(2-p-chlorphenyl-2-chlorovinyl)-3,3-dimethylcycloprop-1-yl)-α-oxacetonitril, Cyclobutancarbonsäurecyanid, Methylcyclobutancarbonsäurecyanid, Dimethylcyclobutancarbonsäurecyanid, Cyclopentancarbonsäurecyanid, Cyclohexancarbonsäurecyanid, Acrylsäurecyanid, Methacrylsäurecyanid, Crotonsäurecyanid, Cyclohexencarbonsäurecyanid, Benzoylcyanid, m-Chloro-benzoylcyanid, p-Chlorobenzoylcyanid, o-Chloro-benzoylcyanid, 2,3-Dichloro-, 3,4-Dichloro-, 3,5-Dichloro-, 2,6-Dichloro-benzoylcyanid, o-, m-, p-Methoxy-benzoylcyanid, o-, m-, p-Trifluoromethyl-benzoylcyanid, o-, m-, p-Trifluoromethoxybenzoylcyanid, o-, m-, p-Nitrobenzoylcyanid, Naphthalincarbonsäurecyanid, 1-Phenyl-5-pyrazolon-3-carbonsäurecyanid, Furancarbonsäurecyanid, Thiophencarbonsäurecyanid, Pyridincarbonsäurecyanid.

Die als Ausgangsstoffe verwendbaren O-Acyl-tartronsäurenitrile der allgemeinen Formel (III) sind bekannt oder können nach an sich bekannten Methoden hergestellt werden (Angew. Chemie, 94, S. 1-86 (1982) und die darin angegebenen Literaturstellen).

Bevorzugt werden Verbindungen der Formel (III) eingesetzt, in der der Rest $R^2$ die bei den Verbindungen der Formel (II) als bevorzugt angegebenen Bedeutungen besitzt.

Als bevorzugte Beispiele für die substituierten O-Acyltartronsäurenitrile der allgemeinen Formel (III) seien im einzelnen genannt:

α-Acetyloxy- α-methyl-malonsäuredinitril,
α-Propionyloxy- α-ethyl- "
α-Butyryloxy- α-propyl- "
α-Pivaloyloxy- α-tert.-butyl- "
α-Stearayloxy- α-heptadecyl- "
α-Palmitinoxy- α-pentadecyl- "
α-Oleyloxy- α-heptadecyl- "
α-Cyclopropionyloxy-cyclopropyl- "
α'-[α- 2-(2,2-Dichlorovinyl)-3,3-dimethylcycloprop-1-ionyloxy ]- α-[α'- 2-(2,2-dichlorovinyl)-3,3-dimethylcyclopropyl ]-malonsäuredinitril,
α-[α'- 2-(2-p-Chlorophenyl-2-chlorovinyl)-3,3-dimethylcycloprop-1-ionyloxy ]-α-[α'- 2-(2-p-chlorophenyl)-3,3-dimethylcyclopropyl ]-malonsäuredinitril,
α-Cyclobutionyloxy- α-cyclobutyl-malonsäuredinitril,
α-Methylcyclobutionyloxy- α-methylcyclobutyl- "
α-Dimethylcyclobutionyloxy- α-dimethylcyclobutyl- "
α-Cyclopentionyloxy- α-cyclopentyl- "
α-Cyclohexionyloxy- α-cyclohexyl- "
α-Benzoyloxy- α-phenyl- "
α-Acryloyloxy- α-vinyl- "
α-Methacryloyloxy- α-( α'-methyl-vinyl)- "

α-Butenoyl-α-propenyl-malonsäuredinitril
α-Cyclohexenoyloxy-α-cyclohexenyl- "
α-(m-Chlorobenzoyloxy)-α-(m-chlorophenyl)-malonsäuredinitril,
α-(o-Chlorobenzoyloxy)-α-(o-chlorophenyl)-malonsäuredinitril,
α-(p-Chlorobenzoyloxy)-α-(p-chlorophenyl)-malonsäuredinitril,
α-(2,3-Dichlorobenzoyloxy)-α-(2,3-dichlorophenyl)-malonsäuredinitril,
α-(3,4-Dichlorobenzoyloxy)-α-(3,4-dichlorophenyl)-malonsäuredinitril,
α-(3,5-Dichlorobenzoyloxy)-α-(3,5-dichlorophenyl)-malonsäuredinitril,
α-(2,6-Dichlorobenzoyloxy)-α-(2,6-dichlorophenyl)-malonsäuredinitril,
α-(o-Methoxy-benzoyloxy)-α-(o-methoxy-phenyl)-malonsäuredinitril,
α-(p-Methoxy-benzoyloxy)-α-(p-methoxy-phenyl)-malonsäuredinitril,
α-(m-Methoxy-benzoyloxy)-α-(m-methoxy-phenyl)-malonsäuredinitril,
α-(o-Trifluoromethylbenzoyloxy)-α-(o-trifluoromethylphenyl)-malonsäuredinitril,
α-(m-Trifluoromethylbenzoyloxy)-α-(m-trifluoromethylphenyl)-malonsäuredinitril,
α-(p-Trifluoromethylbenzoyloxy)-α-(p-trifluoromethylphenyl)-malonsäuredinitril,
α-(o-Trifluoromethoxybenzoyloxy)-α-(o-trifluoromethoxyphenyl)-malonsäuredinitril,
α-(m-Trifluoromethoxybenzoyloxy)-α-(m-trifluoromethoxyphenyl)-malonsäuredinitril,

α-(p-Trifluoromethoxybenzoyloxy)-α-(p-trifluoromethoxyphenyl)-malonsäuredinitril,
α-(o-Nitrobenzoyl)-α-(o-nitrophenyl)-malonsäuredinitril,
α-(m-Nitrobenzoyl)-α-(m-nitrophenyl)-malonsäuredinitril,
α-(p-Nitrobenzoyl)-α-(p-nitrophenyl)-malonsäuredinitril,
α-(Naphthoyloxy)-α-(naphthyl)-malonsäuredinitril,
α-(1-Phenyl-5-pyrazolon-3-oyloxy)-α-(1-phenyl-5-pyrazolon-3)-malonsäuredinitril,
α-(Furanoyloxy)-α-(furanyl)-malonsäuredinitril,
α-(Thiophenoyloxy)-α-(thiophenyl)-malonsäuredinitril,
α-(Pyridinoyloxy)-α-(pyridyl)-malonsäuredinitril.

Die als Ausgangsstoffe verwendbaren Aldehyde und Ketone der Formel (IV) sind bekannt oder können nach an sich bekannten Methoden hergestellt werden.

Bevorzugt werden Verbindungen der Formel (IV) eingesetzt, in welcher

R für Wasserstoff, $C_{1-4}$-Alkyl, das gegebenenfalls durch Halogen wie Fluor, Chlor, Brom, CN, $C_{1-4}$-Alkoxy, $C_{1-4}$-Alkoxycarbonyl substituiert ist oder für Phenyl, das gegebenenfalls durch Halogen wie Fluor, Chlor, Brom, CN, $NO_2$, $C_{1-4}$-Alkoxy, $C_{1-4}$-Halogenalkoxy, $C_{1-4}$-Alkyl, $C_{1-4}$-Halogenalkyl, Methylendioxy, Ethylendioxy, die gegebenenfalls halogensubstituiert sind, steht und

$R^1$ für Wasserstoff, $C_{1-10}$-Alkyl, das gegebenenfalls durch Halogen wie Fluor, Chlor, Brom, CN, $C_{1-4}$-Alkoxy, $C_{1-4}$-Alkoxycarbonyl oder gegebenenfalls halogen-, alkyl-, alkoxysubstituiertes Phenyl

substituiert ist oder für $C_{2-6}$-Alkenyl, das gegebenenfalls durch Halogen wie Fluor, Chlor, Brom, $C_{1-4}$-Alkoxy, susbstituiert ist, oder für $C_{3-7}$-Cycloalkyl, das gegebenenfalls durch $C_{1-4}$-Alkyl, Halogen oder CN substituiert ist und an das ein Benzolring ankondensiert sein kann oder für Phenyl oder Naphthyl, die gegebenenfalls durch Halogen, wie Fluor, Chlor, Brom, CN, $NO_2$, $C_{1-4}$-Alkyl, $C_{1-4}$-Alkoxy, $C_{1-4}$-Halogenalkyl, $C_{1-4}$-Halogenalkoxy, $C_{1-4}$-Alkoxycarbonyl, gegebenenfalls halogensubstituiertes Phenoxy, gegebenenfalls fluor- oder chlorsubstituiertes Methylendioxy oder Ethylendioxy substituiert sind, ferner für 5- bis 6-gliedrige O-, S- oder N-haltige Heterocyclen, die gegebenenfalls durch $C_{1-4}$-Alkyl, Halogen wie Fluor oder Chlor, substituiert sind, ferner für Alkylcarbonyl oder Phenylcarbonyl, steht.

R und $R^1$ können zusammen für einen gegebenenfalls durch Halogen wie Fluor, Chlor, Brom, $C_{1-4}$-Alkyl, CN, Alkoxycarbonyl substituierten Cycloalkylrest mit 3 bis 6 C-Atomen oder Cycloalkenylrest mit 5 bis 6 C-Atomen, an die gegebenenfalls ein oder zwei Benzolringe ankondensiert sind, stehen.

Besonders bevorzugt sind Verbindungen der Formel (IV), in welcher

R für Wasserstoff, Methyl, Ethyl, Trihalogenmethyl, Phenyl, Halogenphenyl, Tolyl steht,

$R^1$ für Wasserstoff, $C_{1-4}$-Alkyl, $C_{2-4}$-Alkenyl, Phenyl, Halogenphenyl, Tolyl, Furfuryl, steht,

R und $R^1$ gemeinsam für Cyclopentan oder Cyclohexan stehen.

Als bevorzugte Beispiele für Aldehyde oder Ketone der allgemeinen Formel (IV) seien im einzelnen genannt: Formaldehyd, Acetaldehyd, Propionaldehyd, Butyraldehyd, Isobutyraldehyd, Chloracetaldehyd, Trichloracetaldehyd, Aceton, Chloraceton, Dichloraceton, Hexachloraceton, Hexafluoraceton, Butanon, 3-Chlorbutanon, 2-Pentanon, 3-Pentanon, 3-Methyl-2-butanon, 2,2-Dimethylpropanal, 3,3-Dimethyl-2-butanon, 1-Chlor-3,3-dimethyl-2-butanon, 1,1-Dichlor-3,3-dimethyl-2-butanon, Heptanal, 2-Ethylhexanal, 2-Undecanon, 10-Nonadecanon, Propenal, Butenal, 2-Butenal, 4-Methyl-3-penten-2-on, 2,4-Hexadienal, 2,3-Butandion, Pentandial, 2,4-Pentandion, 2,5-Hexandion, Cyclopropanon, Cyclobutanon, Cyclopentanon, Cyclohexanon, Methylcyclohexanon, 3,3,5-Trimethylcyclohexanon, Cyclododecanon, 3-Cyclohexen-1-carboxaldehyd, 3,3,5-Trimethyl-2-cyclohexan-1-on, Campher, 2-Cyclohexyliden-cyclohexanon, Benzaldehyd, o-, m-, p-Chlorbenzaldehyd, 2,6-Dichlorbenzaldehyd, Trichlorbenzaldehyd, o-, m-, p-Nitrobenzaldehyd, 1-Phenylethanon, 1-(3-Nitrophenyl)-ethanon, 1-(4-Nitrophenyl)-ethanon, o-, m-, p-Toluylaldehyd, Phenylpropanal, 5-Methyl-1-phenyl-3-hexanon, 3-Phenyl-2-propenon, α-Tetralon, Benzophenon, Fluorenon, Anthron, 1,4-Benzoldicarboxaldehyd, Benzyl, Pryidinaldehyd, Furfural, 3-Phenoxybenzaldehyd, 3-Phenoxy-4-fluorbenzaldehyd.

Als Basen, die erfindungsgemäß eingesetzt werden können, kommen in Frage: Alkali- und Erdalkalihydroxide, Alkalicarbonate, Alkalialkoholate, Alkalisalze schwacher organischer Säuren sowie tertiäre Amine.

Besonders bevorzugt ist NaOH, KOH, Natriummethylat, Natriumethylat, Natriumacetat, Natriumbenzoat.

Weiter wird besonders bevorzugt: Trimethylamin, Triethylamin, Tripropylamin, Tributylamin, Dimethylcyclohexylamin, N-Dimethylanilin, N-Dimethylbenzylamin, N-Methylmorpholin, N-Methylpyrrolidin, N-Methylpiperidin, Pyridin, Picolin, Chinolin, N,N'-Dimethylpiperazin, N-Methyl-N'-(ß-dimethylaminoethyl)-piperazin, 1,4,5,6-Tetrahydro-1,2-dimethylpyrimidin, 1,4-Diazabicyclo-[2,2,2]-octan, 1,5-Diazabicyclo-[4,3,0]-non-5-en, 1,8-Diazabicyclo-[5,4,0]-undec-7-en, Bis-(2-dimethylaminoethyl)-ether.

Als Verdünnungsmittel, die bei der Durchführung des erfindungsgemäßen Verfahrens eingesetzt werden können, kommen alle inerten organischen Lösungsmittel, die nicht mit den Ausgangskomponenten der Formeln (II), (III) und (IV) in eine chemische Reaktion eintreten, in Betracht. Solche Lösungsmittel sind beispielsweise Xylole wie o-Xylol, Chlorobenzol, o-Dichlorbenzol, die Trichlorbenzole, Nitrobenzol, Tetramethylensulfon, Acetonitril, Benzonitril, Glutarsäuredinitril, Ethylenglykoldimethylether, Diethylenglykoldimethylether, Ethylenglykolmethyletheracetat. Prinzipiell ist es jedoch auch möglich, die erfindungsgemäße Umsetzung ohne Verdünnungsmittel durchzuführen.

Die Reaktionstemperatur kann in einem größeren Bereich variiert werden. Im allgemeinen arbeitet man bei Temperaturen zwischen 0 und 250°C, vorzugsweise zwischen 20 und 180°C.

Die Reaktion wird im allgemeinen bei Normaldruck durchgeführt. Bei Verwendung von niedrigsiedenden, aliphatischen Aldehyden oder Ketonen ist jedoch ein leichter Überdruck günstig, im allgemeinen von 1 bis 10, vorzugsweise von 1 bis 5 Atmosphären Überdruck.

Bei der Durchführung des erfindungsgemäßen Verfahrens setzt man im allgemeinen 1 Mol Acylcyanid oder substituiertes O-Acyl-tartronsäurenitril mit der stöchiometrischen Menge Aldehyd bzw. Keton um.

Die Aufarbeitung erfolgt nach beendeter Umsetzung üblicherweise durch Destillation und gegebenenfalls Umkristallisation.

Auch in der Gasphase kann das Gemisch aus Acylcyanid oder substituiertem O-Acyl-tartronsäurenitril mit Aldehyd bzw. Keton in Gegenwart der erfindungsgemäßen Katalysatoren umgesetzt werden.

Weiterhin ist es möglich, das erfindungsgemäße Verfahren kontinuierlich zu gestalten.

Die nach dem erfindungsgemäßen Verfahren leicht zugänglichen Cyanhydrincarbonsäureester sind Ausgangsprodukte z.B. für die Herstellung α,β-ungesättigter Nitrile oder stellen selbst biocide Mittel dar. So ist z.B.

das α-Cyano-3-phenoxybenzyl-2-(2,2-dichlorvinyl)-3,3-dimethylcyclopropan-1-carboxylat insektizid und akarizid wirksam (DE-OS 3 047 881; US-PS 4 234 507).

Die folgenden Beispiele illustrieren das erfindungsgemäße Verfahren ohne eine Begrenzung hinsichtlich seiner Durchführbarkeit anzugeben.

Beispiel 1

In einem 250 ml Kolben werden 52,4 g α-Benzoyloxy-α-phenylmalonsäuredinitril (0,2 Mol) mit 42,4 g Benzaldehyd (0,4 Mol) und 1 g 1,4-Diaza-bicyclo-/2,2,2/-octan gemischt und die Innentemperatur unter Rühren innerhalb von 30 Minuten auf 160°C gesteigert. Es wird 2 Stunden bei dieser Temperatur gehalten, nach dem Erkalten durch Destillation gereinigt.
Ausbeute: 87 g Benzaldehyd-cyanhydrinbenzoat (≙ 92 % der Theorie).
Siedepunkt: 156-159°C bei 0,2 mbar.

Beispiel 2

In einem 150 ml Autoklav werden 52,4 g α-Benzoyloxy-α-phenylmalonsäuredinitril (0,2 Mol) mit 17,6 g Acetaldehyd (0,4 Mol) in Gegenwart von 1 g Natriummethylat 3 Stunden auf 170°C erwärmt. Nach dem Erkalten wird fraktioniert destilliert.
Ausbeute: 65 g Acetaldehydcyanhydrinbenzoat (≙ 93 % der Theorie).
Siedepunkt: 98-100°C bei 0,1 mbar.

Beispiel 3

In einem 250 ml Dreihalskolben mit Rührer, Kühler und Tropftrichter werden 52,4 g α-Benzoyloxy-α-phenylmalonsäuredinitril (0,2 Mol) mit 2 g N-Dimethyl-benzylamin versetzt und auf 170°C erhitzt. Innerhalb von 4 Stunden tropft man 17,6 g Acetaldehyd (0,4 Mol) zu.

Nach beendetem Zutropfen wird fraktioniert destilliert.
Ausbeute: 57 g Acetaldehydcyanhydrinbenzoat (81,5 % der Theorie).
Siedepunkt: 98-100°C bei 0,1 mbar.

Beispiel 4

In einem 250 ml Dreihalskolben mit Rührer, Kühler und Tropftrichter werden 52,4 g α-Benzoyloxy-α-phenylmalonsäuredinitril (0,2 Mol) mit 1 g Natriumbenzoat versetzt und auf 180°C erwärmt. Innerhalb von 5 Stunden werden 28,8 g Isobutyraldehyd (0,4 Mol) langsam zugetropft. Danach wird die Mischung fraktioniert destilliert.
Ausbeute: 64 g Isobutyraldehydcyanhydrinbenzoat (≙ 79 % der Theorie).
Siedepunkt: 95-97°C bei 0,1 mbar.

Beispiel 5

In einem 150 ml Dreihalskolben werden 44,4 g α-Pivaloyloxy-α-tert.-butylmalonsäuredinitril (0,2 Mol) auf 170°C erwärmt, mit 2 g 1,8-Diazabicyclo-[5,4,0]-undec-7-en versetzt und innerhalb von 1 Stunde 28,8 g Isobutyraldehyd (0,4 Mol) zugetropft. Es wird 30 Minuten nachgerührt, anschließend destilliert.
Ausbeute: 63 g Isobutyraldehydcyanhydrinpivaloat (≙ 86 % der Theorie).
Siedepunkt: 98°C bei 16 mbar.

Beispiel 6

In einem 150 ml Kolben werden 44,4 g α-Pivaloxy-α-tert.-butylmalonsäuredinitril (0,2 Mol) mit 1 g 1,5-Diazabicyclo-[4,3,0]-non-5-en und 38,4 g Furfural (0,4 Mol) versetzt und 30 Minuten auf 180°C erwärmt, anschließend wird fraktioniert destilliert.
Ausbeute: 78 g Furfuralcyanhydrin-pivaloat (≙ 94 % der Theorie).
Siedepunkt: 75-77°C bei 0,2 mbar.

Beispiel 7

In einem 150 ml Kolben werden 44,4 g α-Pivaloyloxy-α-tert.-butyl-malonsäuredinitril (0,2 Mol), 12 g Paraformaldehyd (0,4 Mol) und 1 g 1,4-Diazabicyclo-[2,2,2]-octan gemischt und 1 Stunde unter Rühren auf 160°C erwärmt. Nach der Destillation erhält man 52,9 g Formaldehydcyanhydrin-pivaloat (94 % der Theorie).
Siedepunkt: 48-50°C bei 0,8 mbar.

Beispiel 8

In einem 150 ml Kolben werden 131 g Benzoylcyanid (1 Mol) mit 80 g (1 Mol) Methylethylketon und 2 g 1,4-Diazabicyclo-[2,2,2]-octan gemischt und zum Sieden erwärmt. Innerhalb von 5 Stunden steigt die Innentemperatur von 117°C auf 160°C. Das Reaktionsprodukt wird destilliert.
Ausbeute: 140 g Methylethylketoncyanhydrin-benzoat (≙ 69 % der Theorie).
Siedepunkt: 156-159°C bei 15 mbar.

Beispiel 9

In einem Kolben werden 131 g Benzoylcyanid (1 Mol), 98 g Cyclohexanon (1 Mol) und 1 g Natriumbenzoat auf 150°C erwärmt. Das Reaktionsgemisch erwärmt sich von selbst auf 170°C. Es wird nach 30 Minuten bei 165°C nachgerührt, anschließend destilliert.
Ausbeute: 200 g Cyclohexanoncyanhydrinbenzoat (≙ 87,5 % der Theorie).
Siedepunkt: 138-140°C bei 0,3 mbar.

Beispiel 10

In einem Autoklav werden 131 g Benzoylcyanid (1 Mol), 44 g Acetaldehyd (1 Mol) und 1 g Natriummethylat gemischt und 3 Stunden auf 150°C erwärmt. Nach dem Erkalten wird das Reaktionsgemisch destilliert.
Ausbeute: 170 g Acetaldehydcyanhydrinbenzoat (≙ 97 % der Theorie).
Siedepunkt: 138-140°C bei 15 mbar.

Beispiel 11

In einem Kolben werden 131 g Benzoylcyanid (1 Mol), 100 g Benzaldehyd (1 Mol) und 1 g 1,4-Diazabicyclo-/2,2,2/-octan gemischt und auf 100°C erwärmt. Bei dieser Temperatur setzt eine exotherme Reaktion ein. Nach dem Erkalten wird der Rückstand aus Ethanol umkristallisiert.
Ausbeute: 221 g Benzaldehydcyanhydrinbenzoat (≙ 93 % der Theorie).
Siedepunkt: 146-150°C bei 0,2 mbar; Festpunkt: 58°C.

Beispiel 12

In einem Dreihalskolben werden 131 g Benzoylcyanid (1 Mol) vorgelegt, mit 1 g 1,4-Diazabicyclo-2,2,2-octan versetzt und auf 80°C erwärmt. Bei dieser Temperatur tropft man 56 g Acrolein (1 Mol), dem 0,5 g Hydrochinon zugesetzt sind, innerhalb von 30 Minuten zu und rührt 30 Minuten bei 100°C nach.
Nach der Destillation erhält man 116 g Acroleincyanhydrinbenzoat (= 62 % der Theorie).
Siedepunkt: 107-109°C bei 0,2 mbar.

Patentanspruch

Verfahren zur Herstellung von Cyanhydrincarbonsäureestern der allgemeinen Formel (I)

$$R^2-\overset{\overset{\displaystyle O}{\|}}{C}-O-\overset{\overset{\displaystyle CN}{|}}{\underset{\underset{\displaystyle R}{|}}{C}}-R^1 \qquad (I)$$

in welcher

R für Wasserstoff, gegebenenfalls durch Halogen, CN, Alkoxy oder Alkoxycarbonyl substituiertes Alkyl oder Aryl steht,

$R^1$ für Wasserstoff, gegebenenfalls durch Halogen, CN, Alkoxy, Alkoxycarbonyl substituiertes Alkyl, Cycloalkyl oder Alkenyl, sowie für Aryl oder Heteroaryl steht und $R^1$ und $R^2$ gemeinsam für gegebenenfalls durch Halogen, CN, Alkyl substituiertes Alkylen stehen,

$R^2$ für gegebenenfalls substituiertes Alkyl, Cycloalkyl, Aryl oder Heteroaryl steht,

indem man Acylcyanide der Formel (II)

$$R^2-\overset{\overset{\displaystyle O}{\|}}{C}-CN \qquad (II)$$

in welcher

$R^2$ die oben angegebene Formel hat,

oder O-Acyl-tartronsäurenitrile (dimere Acylcyanide) der Formel (III)

$$R^2-\overset{\displaystyle O}{\overset{\|}{C}}-O-\underset{\displaystyle CN}{\overset{\displaystyle CN}{C}}-R^2 \qquad (III)$$

in welcher
$R^2$ die oben angegebene Bedeutung hat,

oder Gemische der Verbindungen der Formel (II) und (III) mit Aldehyden oder Ketonen der Formel (IV)

$$O=\underset{\displaystyle R}{C}-R^1 \qquad (IV)$$

in welcher
$R^1$ und R die oben angegebene Bedeutung haben,

in Gegenwart wasserfreier Basen gegebenenfalls in Gegenwart von Verdünnungsmitteln umsetzt.

Europäisches Patentamt

# EUROPÄISCHER RECHERCHENBERICHT

0093338

Nummer der Anmeldung

EP 83 10 3906

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| Y,D | ANGEWANDTE CHEMIE, Band 94, Heft 1, 1982, Seiten 1-15, Verlag Chemie GmbH., Weinheim, DE. SIEGFRIED HÜNIG et al.: "Zur Chemie der Acylcyanide" * Seiten 1,5,11,13,14 * --- | 1 | C 07 C 120/00<br>C 07 C 121/38<br>C 07 C 121/75 |
| Y,D | THE JOURNAL OF THE AMERICAN CHEMICAL SOCIETY, Band LXXI, Januar-April 1949, Seiten 34-36, Mack Printing Cy., Easton, PA., USA C.S. MARVEL et al.: "Benzoyl cyanide dimer and the addition of benzoyl cyanide to aromatic aldehydes" * Seite 36 * --- | 1 | |
| Y,D | ANGEWANDTE CHEMIE, Band 68, Nr. 13, 7. Juli 1956, Seiten 425-435, DE. J. THESING et al.: "Die Chemie der Acylcyanide" * Seiten 432-434 * ----- | 1 | RECHERCHIERTE SACHGEBIETE (Int. Cl. 3)<br>C 07 C 120/00<br>C 07 C 121/00 |

Der vorliegende Recherchenbericht wurde für alle Patentansprüche erstellt.

| Recherchenort | Abschlußdatum der Recherche | Prüfer |
|---|---|---|
| DEN HAAG | 15-07-1983 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82